# EUROPÄISCHE PATENTSCHRIFT

(11) **EP 0 797 960 B1**
(45) Veröffentlichungstag und Bekanntmachung des Hinweises auf die Patenterteilung: **06.09.2000**
(21) Anmeldenummer: 97110013.6
(22) Anmeldetag: 08.04.1994
(51) Int. Cl.: A61B 17/86

(54) **Pedikelschraube**
pedicle screw
vis pédiculaire

(30) Priorität: 18.05.1993 DE 4316542
(43) Veröffentlichungstag der Anmeldung: 01.10.1997
(62) Teilanmeldung aus: 94913558.6
(73) Patentinhaber: Schäfer, Bernd, 6315 Oberägeri (CH)
(72) Erfinder: Halm, Henry, Dr., 49143 Bissendorf-Wissingen (DE); Rehder, Günther, 73650 Winterbach (DE)
(74) Vertreter: Dreiss, Fuhlendorf, Steimle & Becker, Patentanwälte

(56) Entgegenhaltungen:
- EP-A- 0 282 789
- DE-U- 9 011 312
- DE-U- 9 104 027
- FR-A- 2 642 643
- US-A- 5 120 171

## Beschreibung

Die Erfindung betrifft eine eine Pedikelschraube, mit einem Gabelkopf und einem Schraubenschaft.

Knochenschrauben sind in einer Vielzahl bekannt (DE-Gbm 90 04 240, DE-Gbm 91 04 027, EP-A-346 521, DE-A 39 42 429, EP-A-443 894, EP-A-348 272, EP-A-465 158, EP-A-528 706, EP-A-443 892, DE-A 39 16 198, DE-C 41 10 002). Aus diesen Druckschriften sind unterschiedliche Knochenschrauben mit Schraubenköpfen bekannt, bei denen der Schraubenschaft im wesentlichen zylinderförmig ausgebildet ist.

Aus der DE 91 04 027 U1 ist eine Pedikelschraube mit konischem Gewindequerschnitt bekannt, der in einem Abschnitt des Schraubenschaftes ausgebidet ist. Außerdem weist der Gewindebereich einen konstanten Außendurchmesser auf.

Der Erfindung liegt die Aufgabe zugrunde, eine Pedikelschraube der eingangs genannten Art derart weiterzubilden, daß sie besser im Knochen verankerbar ist.

Diese Aufgabe wird erfindungsgemäß dadurch gelöst, daß die Breite der Gewindeschneide der Pedikelschraube von der Schraubenspitze ausgehend zunimmt.

Die konische Form des Schraubenkerns hat den Vorteil, daß die Pedikelschraube besser im Knochen fixierbar ist, und insbesondere beim Einschrauben in den Knochen stramm sitzt. Die Pedikelschraube wird nicht nur über die Gewindegänge gehalten, sondern auch durch die Klemmwirkung des sich konisch erweiternden Schraubenkerns.

Durch die zunehmende Breite der Gewindeschneide der Pedikelschraube von der Schraubenspitze ausgehend wird eine Klemmwirkung erzielt, durch die die Pedikelschraube zusätzlich am Knochen festgehalten wird. Dabei ist bevorzugt die Gewindesteigung konstant.

Vorteilhaft erweitert sich der Schraubenkern bis zum Außen-bzw. Nenndurchmesser der Pedikelschraube.

Weitere Vorteile, Merkmale und Einzelheiten der Erfindung ergeben sich aus der nachfolgenden Beschreibung, in der unter Bezugnahme auf die Zeichnung ein besonders bevorzugtes Ausführungsbeispiel im einzelnen dargestellt ist. Dabei können die in der Zeichnung dargestellten und in der Beschreibung erwähnten Merkmale jeweils einzeln für sich oder in beliebiger Kombination bei der Erfindung verwirklicht sein. In der Zeichnung zeigen:
- Figur 1: eine Seitenansicht der erfindungsgemäßen Pedikelschraube; und
- Figur 2: eine Seitenansicht in Richtung des Pfeils II gemäß Figur 1, teilweise aufgeschnitten.

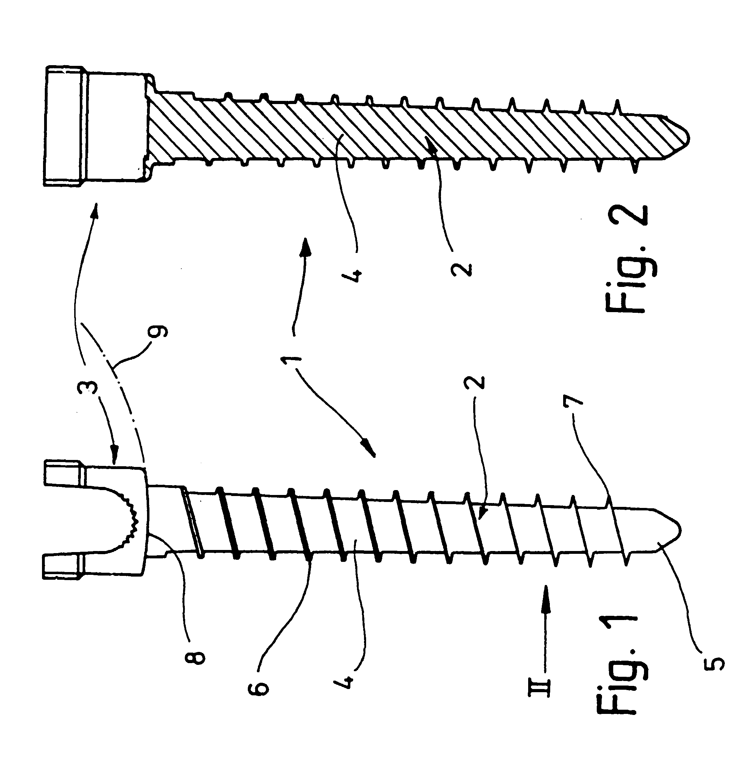

In der Figur 1 ist insgesamt mit 1 eine Pedikelschraube dargestellt, die einen Gewindeschaft 2 und einen Gabelkopf 3 aufweist. Der Gewindeschaft 2 besteht aus einem Schraubenkern 4 mit Schraubenspitze 5 und einem den Schraubenkern 4 umgebenden Gewinde 6. Dabei ist erkennbar, daß der Schraubenkern 4 sich von der Schraubenspitze 5 in Richtung des Gabelkopfes 3 konisch erweitert. Der Durchmesser des Gewindes 6 bleibt jedoch konstant, wohingegen die Breite der Gewindeschneide 7 ausgehend von der Schraubenspitze 5 in Richtung des Gabelkopfes 3 zunimmt. Die Steigung des Gewindes 6 bleibt jedoch über die ganze Länge des Gewindeschaftes 2 konstant. Ferner ist in Figur 1 erkennbar, daß die Unterseite 8 des Gabelkopfes 3 ballig, insbesondere kugelförmig ausgebildet ist, was auch durch die Linie 9 andeutungsweise dargestellt sein soll.

## Patentansprüche

1. Pedikelschraube (1) mit einem Gabelkopf (3) und einem Schraubenschaft, bei der die Pedikelschraube (1) einen sich von der Schraubenspitze (5) in Richtung des Gabelkopfes (3) konisch erweiternden Schraubenkern (4) aufweist und der Außen- bzw. Nenndurchmesser der Pedikelschraube (1) konstant ist, dadurch gekennzeichnet, daß die Breite der Gewindeschneide (7) der Pedikelschraube (1) von der Schraubenspitze (5) ausgehend zunimmt.

2. Pedikelschraube nach Anspruch 1, dadurch gekennzeichnet, daß sich der Schraubenkern (4) bis zum Außen- bzw. Nenndurchmesser der Pedikelschraube (1) erweitert.

3. Pedikelschraube nach einem der vorhergehenden Ansprüche, dadurch gekennzeichnet, daß die Gewindesteigung konstant ist.

## Claims

1. A pedicle screw (1) having fork head (3) and a body wherein the pedicle screw (1) includes a screw body (4) which extends conically from said screw point (5) in direction to said fork head (3) and the outer or nominal diameter of the pedicle screw (1) is constant characterized in that the width of the thread (7) of the pedicle screw (1) is increasing starting from said screw point (5).

2. A pedicle screw according to claim 1, characterized in that the screw body (4) increases up to the outer or nominal diameter of the pedicle screw (1).

3. A pedicle screw according one of the preceding claims, characterized in that the pitch of said thread is constant.

## Revendications

1. Vis pédiculaire (1) avec une chape (3) et une tige de vis, dans laquelle la vis pédiculaire (1) présente un corps de vis (4) s'évasant de manière conique depuis la pointe de vis (5) en direction de la chape (3) et dans laquelle le diamètre externe ou nominal de la vis pédiculaire (1) est constant, caractérisée en ce que la largeur du filet (7) de la vis pédiculaire (1) augmente à mesure que l'on s'éloigne de la pointe de vis (5).

2. Vis pédiculaire selon la revendication 1, caractérisée en ce le corps de vis (4) s'évase jusqu'à atteindre le diamètre externe ou nominal de la vis pédiculaire (1).

3. Vis pédiculaire selon l'une des revendications précédentes, caractérisée en ce que le pas de vis est constant.
